Europäisches Patentamt

European Patent Office

Office européen des brevets

(11) Publication number: **0 398 611**

**A1**

## EUROPEAN PATENT APPLICATION

(21) Application number: **90305157.1**

(22) Date of filing: **14.05.90**

(51) Int. Cl.⁵: **D06N 3/14, D06N 7/00, A61L 15/00**

(30) Priority: **18.05.89 GB 8911409**

(43) Date of publication of application:
**22.11.90 Bulletin 90/47**

(84) Designated Contracting States:
**AT BE CH DE DK ES FR GB GR IT LI LU NL SE**

(71) Applicant: **SLUMBERLAND HOLDINGS LIMITED**
**Victoria House, Victoria Street, Chadderton**
**Oldham OL9 0HB(GB)**

(72) Inventor: **Woodcock, Ashley**
**Glenside, 67 Bankhall Lane**
**Hale, Cheshire WA15 0LN(GB)**

(74) Representative: **Lawrence, John Gordon et al**
**McNeight & Lawrence Regent House Heaton**
**Lane**
**Stockport, Cheshire SK4 1BS(GB)**

(54) **Infection barrier.**

(57) There is disclosed a surgical or hygenic barrier artefact or a filter comprising a fabric operative to prevent the passage of infectious agents through it from one side of the fabric to the other and comprising a substrate and a non-porous coating of the type which transports water only in the vapour phase.

EP 0 398 611 A1

## INFECTION BARRIER

This invention relates to barriers against transmission of infectious agents, such as bacteria, viruses and agents of sub-viral dimensions, for example viroids and prions (such agents hereinafter being known collectively as "infectious agents"). Inter alia, this invention relates to such barriers which can be adapted for use in domestic, laboratory and hospital environments.

Viruses are often thought of as amongst the most metabolically efficient of parasites.

Virion genetic material may be encapsidated or otherwise located within a proteinaceous-lipidaceous shell. The capsids of many pathogenic human and animal viruses are coated with an envelope comprising membrane-type lipids and glycoproteins which are capable of interacting with suitable receptors and binding-proteins in host cell membranes thus facilitating attachment of the virion to a susceptible cell.

Such an attached virion may be endocytosed, or the viral envelope may fuse with the cell membrane of susceptible host cells: in both cases viral nucleic acids are delivered to the interior thereof with attendent viral subversion of host cell replicative and metabolic machinery.

It is clear that, by and large, the pathogenicity of the infectious agent is dependent upon exposure of susceptible cells thereto. Conventional ultra-filtration techniques are ineffective in removing many agents pathogenic to higher organisms (including plants) from air and fluids.

The full extent of human, animal and plant morbidities resulting from contact thereof with infectious agents is not established, but is considerable. Moreover, ingestion, for example, of such agents by virtue of their transfer from contaminated surfaces to the mouth readily takes place, leading to contamination of respiratory and gastrointestinal tracts.

Barriers fully effective against a spectrum of infectious agents, and viruses in particular, exist only by virtue of their being non-porous solid sheets.

The use of such barriers as wound dressings, for example, may slow the rate of wound healing by inducing hypoxia, and can cause exacerbation of infection. Breathable wound dressings, however, which are in common use, are not at all effective as barriers to many infectious agents.

Surgical gowns, drapes and other artefacts such as gloves are either permeable to many infectious agents - so that the surgeon can contaminate, or be contaminated by, the patient - or are of non-porous solid material in which case they are extremely uncomfortable to wear for any extended period. Impermeable gowns and drapes are increasingly important nowadays in view of the incidence of such infectious agents as HIV and the causative agent of BSE. Surgical gloves have long been a problem, being so uncomfortable as to require changing during the course of an operation.

Breathable but water inpenetrable fabrics have been produced for use in, for example, clothing for sailing and other outdoor pursuits, an example being the well-known microporous fabric Gore Tex (Registered Trade Mark) manufactured by W L Gore and Associates UK Ltd of Kirkston Campus, Livingstone, West Lothian in Scotland. This fabric, however, is not proof against viruses and agents smaller than viruses.

It has now been discovered, however, that certain materials, which have the capacity to transmit water in the vapour phase as well as gases soluble therein, are effective barriers to viral agents and it is upon this discovery that the present invention is based.

The present invention comprises a surgical or hygenic barrier artefact or a filter comprising a fabric operative to prevent the passage of infectious agents through it from one side of the fabric to the other and comprising a substrate and a non-porous coating of the type which transports water only in the vapour phase.

The substrate may be coated on one side so that a material may be provided which approximates more to uncoated fabric in, for example, its handle.

The artefact may comprise inter alia a wound dressing, a screen, a surgeon's or the like gown, a glove, a hat of the kind used by surgeons and nurses in an operating theatre, a face mask or an operating-theatre drape.

The coating may be a hydrophilic polyurethane, such as that provided by the commercially available polyurethane system known as Baxenden Witcoflex 971/973 manufactured by Baxenden Chemicals Limited of Union Lane, Droitwich, Worcestershire in England. Others are described in the literature such, for example, as British Patent Specification No. 1,341,325.

The substrate may comprise inter alia a closely woven nylon, polyester, or other synthetic or natural fibre.

The invention will be further apparent from the following description, with reference to the several examples which are illustrative of a barrier embodying the invention.

In the examples the substrate is coated with a hydrophilic polyurethane system, such as Baxenden Witcoflex 971/973, on one side only to yield an artefact, in this case a surgeon's gown, having uncoated and coated sides, both of which have been treated with infectious agents such as bacteria and viruses to test transmission thereof across the artefact.

A surgeon's gown is merely representative of the range of artefacts or filters of the present invention.

The material so coated approximates well to uncoated fabric with regard to its handle.

In examples 1 to 3, the impermeability to penetration by Pseudomonas aeruginosa (chosen having regard to its readily identifiable and representative characteristics) of a sheet of cotton coated with the Baxenden Witcoflex 971/973 polyurethane system (hereinafter referred to as the fabric) and derived from a surgeon's gown was studied.

In order to simulate the conditions which the fabric might be subjected to under normal use, it was tested in an untreated state; and in treated states as follows: after it had been autoclaved at 121°C for 15 minutes; and after it had been washed either in hypochlorite at a dilution of 1/256, or in an automatic wash programme comprising two washes in a biological detergent, three rinses and a treatment with a fabric conditioner.

Example 1

Samples of the untreated and treated fabrics (as related above) were bound tightly to glass tambours which were placed on blood agar plates and then flooded for 15 minutes with a suspension of P. aeuruginosa at 100 - 1000 cfu/ml. The blood agar plates were then incubated aerobically for 18 hours at 37°C. All culture plates on which the tambour had been placed remained sterile.

Example 2

Discs of the treated and untreated fabrics were placed on blood agar plates and an attempt was made to inoculate the blood agar therethrough by vigorously rubbing the surface with a cotton swab dipped in the suspension of P. aeruginosa (as above). The discs were removed and the plates were incubated aerobically at 37°C for 18 hours. None of the culture plates subjected to an attempted inoculation through the fabric samples exhibited Pseudomonal growth.

Example 3

Discs of the treated and untreated fabrics were placed in a Seitz filter holder and the filter chamber charged with a suspension of P. aeruginosa. The filter assembly was subjected to a negative pressure of 25 inches of mercury for 60 minutes. The filtrate so produced was subcultured onto blood agar plates which were incubated aerobically for 18 hours at 37°C. The plates thus incubated remained sterile.

These results demonstrate that the untreated, and variously treated fabrics were completely impervious to the passage of bacteria, notwithstanding washing in bleach or biological washing powder or autoclaving.

Example 4

A fabric sample, likewise derived from a surgeon's gown, was used for determining the resistance thereof against viral penetration. A recently obtained isolate of poliovirus type two (serologically confirmed) was grown in cultured human embryonic fibroblasts to "high", but undetermined, titre.

About 0.1ml of the thus obtained isolate was placed on one side of the fabric. Swabs were taken from both sides of the fabric at serial times of up to ninety minutes therafter. The swabs were inoculated on to cultures of human embryonic fibroblasts and test virus subsequently recovered therefrom was identified serologically with polio-virus type 2 specific neutralising serum.

Appropriate controls consisted of placing sterile balanced salt solution instead of the viral suspension onto the fabric.

Virus could only be recovered and identifed as above when the swabs were taken from the side of the fabric which initially had the isolate applied to it.

No virus was isolated from swabs of the fabric which were treated with the sterile salts solution. An incubation of up to ninety minutes was chosen since at times greater than this the inoculate tended to dry on the fabric surface.

It will be appreciated that the water vapour transmission properties of barriers made according to the invention provide for such barriers contacting epidermal surfaces enabling the transmission of gases therefrom and thereto by chemisorption along a diffusion gradient. This provides for a breathable fabric barrier against infectious agents suitable for use in hygenic situations, where previously no such barrier was available. A wound dressing comprising such a barrier, at least in part, is thereby provided. Conventional wound dressings, which are breathable thus recognising the desirability of gaseous exchange between the wound surface and the environment do not constitute a barrier to infectious agents.

Where the barrier forms a medical fabric and is comprised, for example, by a wound dressing contacting a wounded epidermal surface, the net direction of flow of moisture through the barrier will be from the said surface. Advantageously moisture diffusion shells will form above the external face of the barrier which will serve further to remove infectious agents from the vicinity of the epidermal surfaces adjacent the said surface.

Previous suggested uses of material of the general type of barrier according to the present invention have included its use in bedding fabrics intended for increased comfort in use by permitting body generated water vapour to be dispersed through the fabrics whilst also acting to prevent liquid from entering the mattress interior, a purpose clearly of value in hospitals and in cases where incontinence is a problem, and for the control of bedsores. In the hospital context a material which facilitates the transport thereacross of water vapour but prevents the transport thereacross of water in the aqueous phase is very useful since this enables effective disinfection of articles encased in such material without such articles becoming wet.

The present invention provides hitherto unanticipated uses for such material in the form of barriers to infectious agents. For such material to be effective as a component of said barrier, however, the material must be completely non-porous, and this requires more careful quality control and testing during its manufacture than hitherto has been assumed satisfactory for previous suggested uses. Such high quality material as envisaged for the present applications is thus novel in this context. Moreover, the artefacts of the invention must be made so that the completed artefact does not exhibit stitch and like-holes through which infectious agents could pass.

It will be appreciated that it is not intended to limit the invention only to the surgeon's gown-derived fabric cited in the above examples, many variations, such as might readily occur to one skilled in the art, being possible, without departing from the scope thereof as defined by the appended claims.

Thus, although the examples describe particularly a fabric derived from a surgeon's gown made according to the invention, and demonstrate its efficacy with regard to preventing transfer of a specific bacterium and virus, the barrier could equally well comprise a wound dressing, a screen, a surgeon's or the like glove, an operating-theatre drape, an upholstery cover, for example a cover for a pillow or a mattress which prevents passage of infectious agents through it.

The efficacy of the barrier has been exemplified with respect to a type two polio virus and the Pseudomonas aeruginosa bacterium, although it should be stressed that the barrier is effective against the transfer thereacross of most, if not all, prokaryotes.

Based on a theoretical consideration of the properties of the coating, it must be assumed that the barrier is effective against infectious agents of sub-viral dimensions.

Although the substrate which is coated with the polyurethane system in the examples is cotton, the choice of substrate is not particularly limited with respect to other than its weave.

## Claims

1. A surgical or hygenic barrier artefact or a filter comprising a fabric operative to prevent the passage of infectious agents through it from one side of the fabric to the other and comprising a substrate and a non-porous coating of the type which transports water only in the vapour phase.

2. A barrier artefact according to claim 1, characterised in that the substrate is coated with a hydrophilic polyurethane system.

3. A barrier according to the preceding claim, characterised in that the polyurethane system is the Baxenden Witcoflex 971/973.

4. A barrier according to any preceding claim, characterised in that the substrate is coated on one side only.

5. A barrier artefact according to any preceding claim, characterised in that the artefact is selected from the group consisting of a wound dressing, a screen, a surgeon's or the like gown, a glove, a hat of the kind used by surgeons and nurses in an operating theatre, a face mask, an operating-theatre drape and an upholstery cover.

6. A barrier according to any preceding claim, characterised in that the substrate is selected from the group consisting of closely woven natural and synthetic fabrics.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8505322 (THORATEC LABORATORIES)<br>* page 13, line 30 - page 15, line 4; claims 1-6 * | 1, 2, 4-6 | D06N3/14<br>D06N7/00<br>A61L15/00<br>A61L31/00 |
| X | WO-A-8505373 (THORATEC LABORATORIES)<br>* page 14, lines 29 - 36; claims 1-40 * | 1, 2, 4-6 | A41D31/02 |
| X | EP-A-52915 (SHIRLEY INSTITUTE)<br>* page 5; claims 1, 20 * | 1, 2, 4-6 | |
| X | EP-A-73948 (H. VON BLUCHER)<br>* page 2, lines 17 - 20; claims 1, 10 *<br>* page 4, lines 16 -28; example 5 * | 1, 2, 6 | |
| D,A | GB-A-1341325 (J. TRACEY SCALES)<br>* page 3, lines 1 - 27; claims 1, 3, 5-8 * | 1-6 | |
| | | | **TECHNICAL FIELDS SEARCHED (Int. Cl.5 )**<br><br>D06N<br>A61L<br>A41D<br>B32B |

The present search report has been drawn up for all claims

| Place of search | Date of completion of the search | Examiner |
|---|---|---|
| THE HAGUE | 20 SEPTEMBER 1990 | PFANNENSTEIN H. |

EPO FORM 1503 03.82 (P0401)